# EUROPÄISCHE PATENTSCHRIFT

(11) **EP 1 345 979 B1**
(45) Veröffentlichungstag und Bekanntmachung des Hinweises auf die Patenterteilung: **14.07.2004**
(21) Anmeldenummer: 01990396.2
(22) Anmeldetag: 08.11.2001
(51) Int. Cl.: C08G 63/60

(54) **NEUE POLYESTER, VERFAHREN ZU IHRER HERSTELLUNG UND AUS DEN POLYESTERN HERGESTELLTE DEPOT-ARZNEIFORMEN**
NOVEL POLYESTERS, METHOD FOR PRODUCING THE SAME AND DEPOT MEDICAMENTS PRODUCED FROM THESE POLYESTERS
NOUVEAUX POLESTERS, LEUR PROCEDE DE FABRICATION ET MEDICAMENTS RETARD OBTENUS A PARTIR DE CES POLYESTERS

(30) Priorität: 10.11.2000 DE 10055742
(43) Veröffentlichungstag der Anmeldung: 24.09.2003
(73) Patentinhaber: SCHWARZ PHARMA AG, 40789 Monheim (DE)
(72) Erfinder: LI, Youxin, 40764 Langenfeld (DE); MOHR, Detlef, 88400 Biberach (DE); SEIFFERT, Tim, 42697 Solingen (DE)
(86) Internationale Anmeldenummer: PCT/EP2001/012888
(87) Internationale Veröffentlichungsnummer: WO 2002/038646

(56) Entgegenhaltungen:
- US-A- 5 929 196

## Beschreibung

### Kurzbeschreibung der Erfindung

Die Erfindung betrifft neue resorbierbare Polyester, hergestellt durch Ringöffnungs-Polymerisation von Hydroxycarbonsäuren in Gegenwart eines elektrolythaltigen Polyols im Extruder.

### Hintergruna der Erfindung

Für die Behandlung chronischer Erkrankungen oder für dauerhafte Substitutions- oder Prophylaxetherapien besteht ein großer Bedarf an Depotformulierungen, die in der Lage sind, einen Arzneistoff über einen längeren Zeitraum in therapeutisch relevanter Weise freizusetzen.

Solche Depotforrnulierungen können auf verschiedene Weise, wie z.B. oral, topisch, inhalativ oder parenteral verabreicht werden.

Für alle Wirkstoffe, für die sehr lange andauernde, gleichbleibende Wirkstoffkonzentrationen erwünscht sind, sind parenterale Depots, z.B. zur subkutanen oder intramuskulären Verabreichung geeignete Arzneiformen, von besonderem Interesse. Dies ist insbesondere dann der Fall, wenn der pharmazeutisch aktive Wirkstoff bei enteraler Gabe nicht ausreichend resorbiert oder abgebaut wird. Solche parenterale Arzneiformen können beispielsweise als Mikropartikel oder Implantate formuliert werden. Mikropartiket sind von besonderem Interesse, da sie auf Grund ihrer geringen Größe kanülengängig sind und einfach appliziert werden können.

Ein Hauptproblem bei der Herstellung geeigneter Depotformen, insbesondere von Wirkstoffen mit höherem Molekulargewicht wie Peptide oder Proteine ist, das das Freisetzungsprofil häufig diskontinuierlich oder mehrphasig ist. Um dies zu vermeiden, wird nach Möglichkeiten gesucht, das Freisetzungsprofil von Peptiden zu beeinflussen. Während die Herstellung der Depotformen, zB. der Mikropartikel, nur eine begrenzte Auswahl an Einflußmöglichkeiten bietet, können die Eigenschaften der den Depotformen signifikant durch die Modifikation der den Depotformen zugrunde liegenden Polyester moduliert werden.

Bei der Herstellung geeigneter Depotformen sind bioabbaubare Polymere von besonderem Interesse. Pharmakologisch aktive Wirkstoffe werden in diesen Polymermatrices eingebettet und ihre Freisetzung durch die Diffusionsgeschwindigkeit im Polymer sowie die Polymerabbaurate reguliert. Die Eigenschaften des Polymers hinsichtlich der Wirkstofffreisetzungs- und Abbaurate bestimmen somit ganz wesentlich die Qualität des pharmazeutischen Depots.

Bioabbaubare Polyester für Wirkstoffeinbettungen auf Basis von Hydroxycarbonsäuren wurden bereits 1973 in der US 3,773,919 beschrieben. Dabei wurden insbesondere Polyester aus Milch- und Glykolsäure vorgeschlagen, die nach in vivo Applikation zunächst nicht-enzymatisch zu Monomeren hydrolysiert und dann vollständig zu CO₂ und H₂O verstoffwechselt werden.

Die beschriebenen Polyester haben jedoch bei Verwendung in Depotformen, insbesondere Mikropartikeln, den Nachteil einer anfänglich starken Wirkstoffanflutung ("burst") durch Auswaschung des oberflächengebundenen Wirkstoffs, die gefolgt ist von einer Phase stark verminderter Freisetzung, ehe eine vom Polyestermassenabbau getragene Wirkstofffreisetzung einsetzt. Da in der Regel eine möglichst lineare Wirkstofffreisetzung gewünscht ist, sind die in der US 3,773,919 beschrieben Polyester nur sehr bedingt zur Herstellung von Depotformen geeignet.

Ein weiteres Problem der in der US 3,773,919 beschriebenen Lactidglycolid-Polyester (PLG-Polyester) ist die langsame Abbaurate. Bei der Herstellung häufig entstehende hochmolekulare Polyester werden nur langsam abgebaut und können zur Akkumulation sowohl von Polyesterresten als auch zu eingeschlossenem Wirkstoff in den Depots, z.B. unter der Haut oder im Muskel führen.

Aus diesem Grund ist es von besonderer Bedeutung, ein bioabbaubares Matrixmaterial zu entwickeln, das so einstellbar ist, dass es in vivo über einen gewünschten Zeitraum zu einer linearen Wirkstofffreisetzung führt und gleichzeitig weitgehend hydrolysiert wird.

Eine Möglichkeit, die Freisetzungsrate und die Abbaurate des korrespondierenden Depots zu steuern, ist es, das Molekulargewicht des Polyesters zu reduzieren.

EP 058 481 beschreibt die Verwendung eines Gemisches aus PLG-Polyestern mit verschiedenen Molekulargewichten. Das Verfahren führt jedoch zu einem sehr heterogenen, schwierig verarbeitbaren Polyestergemisch, dass zur Herstellung von wirkstoffhaltigen Mikropartikeln mittels Sprühtrocknung nur wenig geeignet ist.

EP 299 730 beschreibt die Herstellung eines biokompatiblen Polyesters durch Ringöffnungs-Polymerisation von D,L-Lactid und Glycolid, wobei die Kettenlänge durch Zugabe von Milchsäure gesteuert wird. Die aus den resultierenden PLG-Polyestem hergestellten Depotformulierungen weisen jedoch eine stark sigmoide Wirkstofffreisetzung auf und haben zudem schlechte mechanische Eigenschaften, die eine Verarbeitung zu Mikropartikein erschweren.

EP 407 617 beschreibt die Herstellung eines PLG-Polyesters mit erhöhter Hydrolysegeschwindigkeit durch RingöffnungsPolymerisation von D,L-Lactid und Glycolid. Die Modulation erfolgt durch Zugabe von Glycosiden, die bei der Polymerisationsreaktion an das repetitive Glycolid-Lactid Rückgrad gebunden werden. Auf diesem Polyestern basierenden Depotformen werden zwar vorgeschlagen, jedoch nicht offenbart.

EP 372 221 beschreibt die kontinuierliche Herstellung resorbierbarer Polyester in einem Extruder. Die Herstellung von Polyestern, die zur Verabreichung von Arzneistoffen geeignet sind, wird zwar vorgeschlagen, nicht aber offenbart. Zur Herstellung der jeweils gewünschten Viskositätseigenschaften der Polyester werden "geeignete Zusätze" empfohlen. Allerdings fehlt jeder Hinweis auf die Beschaffenheit dieser Zusätze.

Es war daher die Aufgabe der vorliegenden Erfindung, einen resorbierbaren Polyester zur Verfügung zu stellen, das eine hohe Wirkstoffbeladung ermöglicht, in einer pharmazeutischen Depotform zu einer linearen Wirkstofffreisetzung führt und in einer Rate abgebaut wird, die der Wirkstoffreisetzung angepasst ist, so daß keine Akkumulation des Polyesters im Körper erfolgt.

Eine weitere Aufgabe der Erfindung war es, die mit der Herstellung resorbierbarer Polyester im Batchverfahren verbundenen Upscaling-Probleme zu lösen. Es ist bekannt, dass sich in kleineren Syntheseansätzen gewonnene Resultate in vergrösserten Syntheseansätzen aus thermodynamischen Gründen nicht oder nur unzureichend reproduzieren lassen.

Die Aufgabe wurde erfindungsgemäß gelöst durch die Herstellung resorbierbarer Polyester durch Zusatz von elektrolythaltigen Polyolen, insbesondere Dextransulfat, im Extruder.

### Abbildungen:

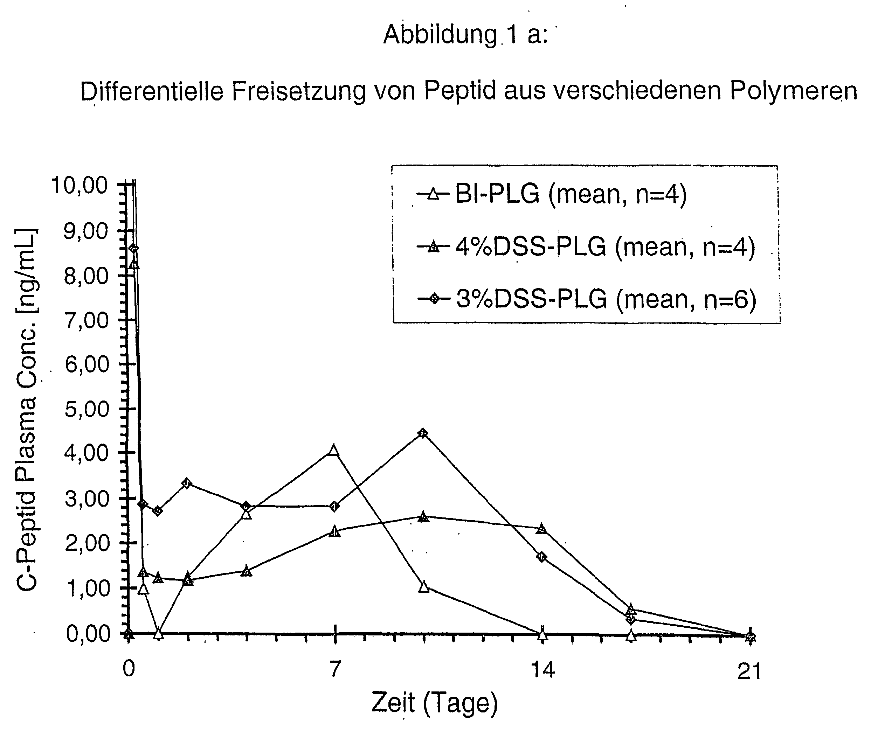

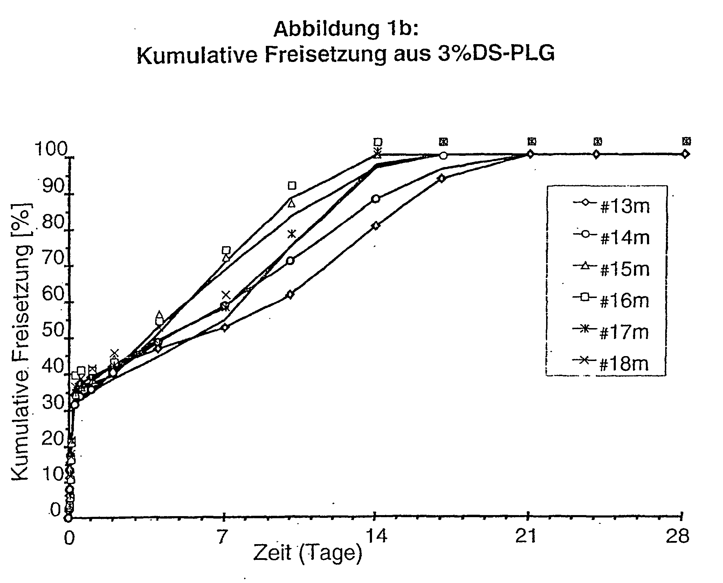

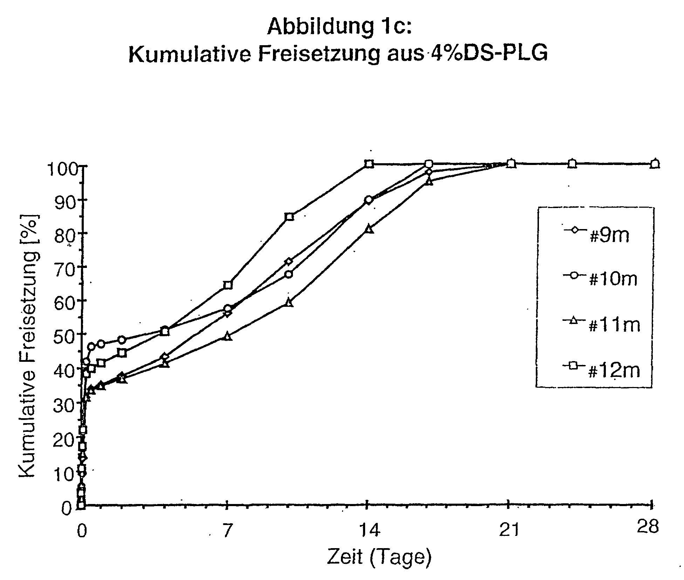

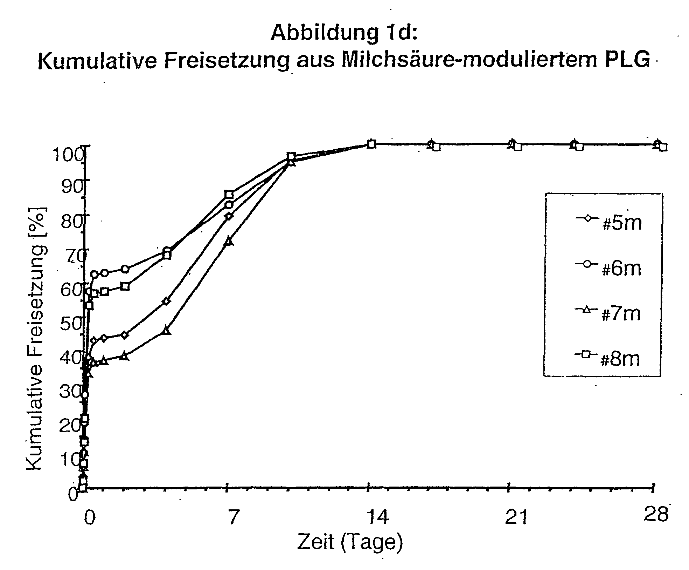

Die Abbildungen 1 zeigen in-vivo Freisetzungsprofile (in Beagle-Hunden) von Mikropartikeln, die auf DS-modifizierten PLG-Polyestern bzw. auf kommerziell erhältlichem PLG-Polyester RG 502 beruhen, wobei die Polyester jeweils mit etwa 7,5% C-Peptid beladen wurden. Abbildung 1a zeigt einen Mittelwert der C-Peptid Plasmakonzentrationen in vier (4% DS; RG 502H), bzw. sechs (3%DS) Hunden. Die Abbildungen 1b-1d zeigen kumulative Freisetzungsmengen von C-Peptid in einzelnen Hunden nach Gabe von 3%DS-PLG (Abb. 1b), 4%DS-PLG (Abb.1c) und käuflichem RG502H (1d).

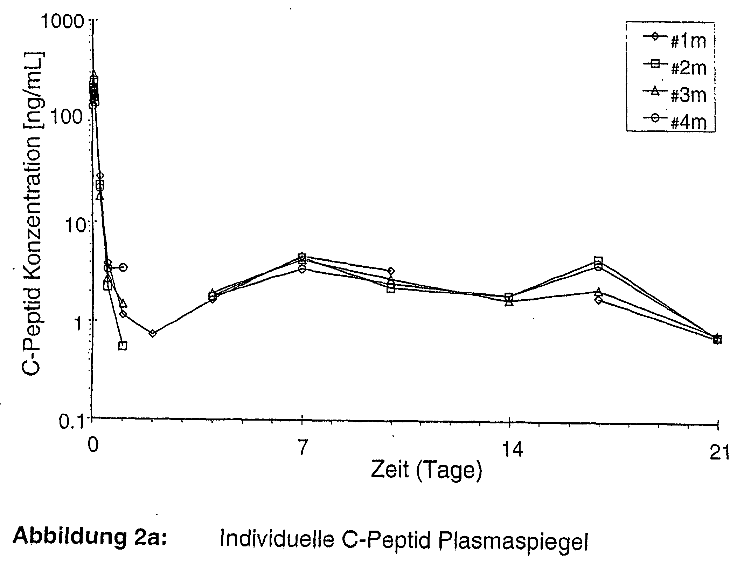

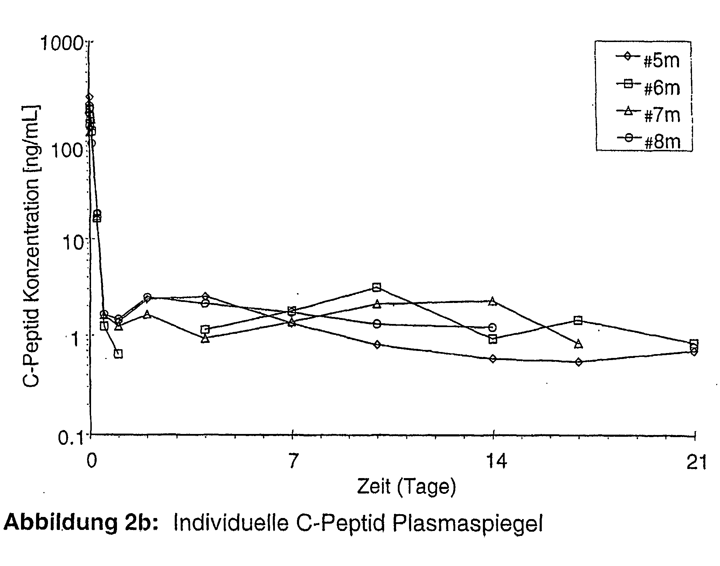

Die Abbildungen 2 zeigen in vivo Freisetzungsprofile (in Beagle-Hunden) von Mikropartikeln, die jeweils mit etwa 7,5% C-Peptid beladen wurden und deren Matrix aus DS-modifizierten PLG-Polyestern besteht. Abbildung 2a zeigt vier individuelle Freisetzungsprofile von mit 3% DS-modulierten Polyestern, die unter Einsatz von 1200 ppm Sn hergestellt wurden, Abbildung 2b zeigt Freisetzungsprofile von mit 3% DS-modulierten Polyestem, die unter Einsatz von 1600 ppm Sn hergestellt wurden

### Beschreibung der Erfindung:

Es zeigte sich überraschenderweise, dass der Zusatz von elektrolythaltigen Polyolen, insbesondere von Dextransulfat (DS), bei der Ringöffnungs-Polymerisation von Hydroxycarbonsäuren, insbesondere von Glycolid und Lactid, zu neuen resorbierbaren Polyestern mit einem über einen längeren Zeitraum weitgehend linearem Freisetzungsprofil führt.

Ein Gegenstand der Erfindung sind daher resorbierbare Polyester, die durch Ringöffnungs-Polymerisation von Hydroxycarbonsäuren in Gegenwart eines elektrolythaltigen Polyols hergestellt wurden.

Unter dem Ausdruck "Polyester" werden in dieser Patentanmeldung sowohl Homopolymere als auch Copolymere, die aus der Polymerisation verschiedener Hydroxycarbonsäuren entstehen, subsumiert.

Unter dem Ausdruck "Polymerisation" wird in dieser Patentanmeldung sowohl die Polymerisation gleicher Monomere, z.B. Lactid, als auch die Copolymerisation verschiedener Monomere, wie z.B. Lactid und Glycolid verstanden.

Unter "Monomeren" werden in dieser Patentanmeldung die unpolymerisierten Bausteine von Polyestern verstanden, daß heißt, auch Hydroxycarbonsäure-Dimere in Lactonform werden hier als "Monomere" bezeichnet.

Unter dem Ausdruck "Hydroxycarbonsäuren" werden in dieser Patentanmeldung auch dimerische Hydroxycarbonsäuren wie Lactid und Glycolid bzw. Hydroxycarbonsäuren in Lactonform subsumiert.

Unter "Lactid" wird in dieser Patentanmeldung L-Lactid, D-Lactid, D,L-Lactid und meso-Lactid subsumiert.

Unter "Moderator" wird in dieser Patentanmeldung ein Stoff verstanden, der bei der Herstellung eines resorbierbaren Polyesters vor dem Polymerisationsprozess zugesetzt wird und der das Molekulargewicht und die Abbaurate des Polyesters und die Freisetzungseigenschaften entsprechender Formulierungen beeinflusst, ohne als eigenständiger Polymerisationskatatysator zu wirken und ohne in substanzieller Menge in das Polyester eingebaut oder daran gebunden zu werden.

Unter "substanzieller Menge" wird in dieser Patentanmeldung ein Anteil von über 200 ppm bezogen auf den Monomeren-Gesamtansatz verstanden.

Unter dem Begriff "Formulierung" wird in dieser Patentanmeldung jede Zubereitung verstanden, die ein Polymer und mindestens einen pharmakologisch aktiven Wirkstoff umfasst.

Durch die Variation des Gehalts der elektrolythaltigen Polyole im Polymerisationsgemisch können das Molekulargewicht und die Verarbeitungseigenschaften des Polyesters, aber auch die Abbaubaurate und das Freisetzungsprofil der auf diesen Polyestern beruhenden Depotformen in besonders einfacher und vorteilhafter Weise eingestellt werden.

Allerdings treten beim Upscaling der ringöffenenden Polymerisation von Hydroxycarbonsäuren im Batchverfahren in Gegenwart von elektrolythaltigen Polyolen, insbesondere von Dextransulfat, bisweilen Probleme auf. So lässt sich die Temperaturführung nach Zugabe von Katalysator und Dextransulfat in großen Ansätzen nur unzureichend kontrollieren, was zu nicht ausreichend reproduzierbaren Resultaten führt.

Zudem ist die ungezügelte, stark exotherme Reaktion auch im Hinblick auf die Zersetzungsneigung und Explosivität mancher elektrolythaltiger Polyole, wie z.B. Dextransulfat, äußerst problematisch, so daß die Prozessführung im Batchverfahren aus Sicherheitsgründen mit erhöhtem apparativen Aufwand verbunden ist.

Demgegenüber wurde von den Erfindern überraschenderweise gefunden, dass die Ringöffnungs-Polymerisation von Hydrocarbonsäuren, insbesondere von Lactid und Glycolid, in Gegenwart von elektrolythaltigen Polyolen in einem Extruder reproduzierbar und sicher zu den erfindungsgemäßen Polyestern führt, die in Depotformen ausgezeichnete Verarbeitungs- und Freisetzungseigenschaften haben.

Extruder, wie zB. Doppelschneckenextruder, sind aus dem Stand der Technik bekannt und werden in der Industrie meist zum Homogenisieren von Granulaten verwendet. Der zur Herstellung der erfindungsgemäßen Polyester verwendete Extruder hat Einfüllvorrichtungen, in die die Monomere oder Monomerengemische, der Modulator, der Polymerisationskatalysator und etwaige Hilfsstoffe eingebracht werden können. Das Polymerisationsgemisch wird sodann entlang der Longitudinalachse des Extruders befördert, wobei ein oder mehrere separat regulierbare Heizelemente passiert werden, die für ein genau einstellbares Temperaturprofil sorgen. Ferner enthält das erste Temperaturelement des Extruders zweckmäßigerweise eine Kühlung, um eine zu starke initiale Erwärmung des Reaktionsgemisches zu verhindern.

Durch die Durchführung der Polymerisation unter Zwangsförderung des Polymerisationsgemisches bei vorgegebenem Temperaturprofil ist somit die Durchführung eines kontinuierlichen Verfahrens mit exakt reproduzierbaren Parametern möglich, was erhebliche Vorteile gegenüber dem diskontinuierlichen, im Upscaling nur sehr schwer reproduzierbaren Batchverfahren aufweist.

Im Hinblick auf den Zusatz elektrolythaltiger Polyole, insbesondere von DS, erlaubt der Einsatz des Extruders zudem die sichere Herstellung des erfindungsgemäßen Polyesters, da im Gegensatz zum Batchverfahren kontinuierlich nur kleine Substanzmengen umgesetzt werden. Somit tritt im Vergleich zum Batchverfahren keine starke initiale Temperaturentwicklung auf. Zudem gewährleisten die Entgasungszonen des Extruders eine Drukkentlastung.

Ein Gegenstand der Erfindung ist daher ein Polyester, hergestellt durch die Ringöffnungs-Polymerisation von Hydroxycarbonsäuren in Lactonform in Gegenwart eines elektrolythaltigen Polyols im Extruder. Diese Polyester werden in dieser Patentanmeldung auch als "Polyol-modulierte Polyester" bezeichnet.

Geeignete Hydroxycarbonsäuren sind beispielsweise Lactid, Glycolid, Methlyglycolid, Dimethylglycolid, Diethylgylcolid, Dibutylglycolid, Caprolacton, Valerolacton, Propiolacton, Butyrolacton und Pivalolacton.

Besonders bevorzugt werden Polyester, hergestellt durch die Ringöffnungs-Polymerisation von Lactid und Glycolid in Gegenwart eines elektrolythaltigen Polyols im Extruder. Diese Polyester werden in dieser Patentanmeldung auch als "Polyol-modulierte PLG-Polyester" bezeichnet.

Ganz besonders bevorzugt werden Polyester, hergestellt durch die Ringöffnungs-Polymerisation von Lactid und Glycolid in Gegenwart von DS im Extruder. Diese Polyester werden in dieser Patentanmeldung auch als "DS-modulierte PLG-Polyester" oder "DS-PLG-Polyester" bezeichnet.

Bei den erfindungsgemäßen Polyestern wurde das elektrolythaltige Polyol nicht oder nur in sehr geringer Menge eingebaut So ist der Schwefelgehalt der Polyester nach Herstellung im Extruder unter DS-Zusatz regelmäßig unterhalb der Nachweisgrenze von 5-10 ppm, und auch der Kohlenhydratanteil ist mit unter 200 ppm unerwartet gering. Daraus ergibt sich, das, wenn überhaupt, nur sehr wenige Teitfraktionen des Polyesters mit Sulfat oder Kohlenhydraten modifiziert sind.

Die durch das erfindungsgemäße Verfahren hergestellten DS-modulierten PLG-Polyester weisen gegenüber den aus dem Stand der Technik bekannten PLG-Polyester (z.B. die kommerziell erhältlichen Polyester RG502H und RG 503H; Boehringer Ingelheim) mit gleichem Molekulargewicht ein deutlich verbessertes Freisetzungsprofil und damit überlegene Eigenschaften auf (siehe Ausführungsbeispiel 6, Abbildungen 1-2). Ein Grund hierfür sind zum einen die besonders gut einstellbaren und kontinuierlichen Prozessbedingungen im Extruder sowie zum anderen der Einfluss des elektrolythaltigen Polyols.

Ohne an eine bestimmte Theorie gebunden sein zu wollen, kann vermutet werden, dass es neben der Hauptfraktion, die aus chemisch unmodifizierten Polyhydroxycarbonsäure-Strukturen besteht, zusätzlich Spurenanteile leicht modifizierter Polyester gibt, die analytisch nicht direkt charakterisierbar sind, die aber das Verhalten der Gesamtfraktion beinflussen.

Elektrolythaltige Polyole, insbesondere Dextransulfat, können somit als Moderatoren des erfindungsgemäßen Herstellungsprozesses aufgefasst werden.

Ein bevorzugter Gegenstand der Erfindung ist daher ein Polyester, hergestellt durch die Ringöffnungs-Polymerisation von Hydroxycarbonsäuren in Lactonform in Gegenwart eines elektrolythaltigen Polyols im Extruder, wobei besagter Polyester im wesentlichen von elektrolythaltigen Polyolen freie Hydroxycarbonsäure-Einheiten enthält.

Besonders bevorzugt werden erfindungsgemäß Polyol-modulierte PLG-Polyester, die aus im wesentlichen von elektrolythaltigen Polyolen freien Lactid und Glycolid-Einheiten aufgebaut sind.

Unter dem Ausdruck "im wesentlichen frei von elektrolythaltigen Polyolen" wird in dieser Patentanmeldung verstanden, dass das elektrolythaltige Polyol, das als Moderator zugesetzt wird, zu einem Anteil von höchstens 200 ppm in das Polyester eingebaut wird.

Als Elektrolytsubstituenten des Moderators können dabei beispielsweise Sulfate, Phosphate, Carboxylgruppen oder Aminogruppen dienen, die an Polyole gebunden sind.

Der Polyolanteil des Moderators kann aus gleichen oder verschiedenen miteinander verknüpften Einheiten aufgebaut sein und lineare oder zyclische Struktur aufweisen. Beispiele hierfür sind Polymere oder Oligomere aus Kohlenhydraten wie Inuline, Dextrane, Xylane, Cyclodextrine sowie mit Hydroxygruppen substituierte Polyester wie Polyvinylalkohol (PVA) und schließlich Copolymere aus PVA mit z.B. Acrylsäure.

Als Moderatoren bevorzugte elektrolythaltige Polyole sind Dextransutfat, Diethylaminoethyl-Dextran, Xylansutfat, Cyclodextrinsulfat und teilsutfatierter PVA, wobei Dextransulfat (DS) besonders bevorzugt wird.

Geeignet ist dabei sowohl Dextransulfat mit hohem (MG 500 kDa) als auch mit mittlerem (8-30 kDa) Molekulargewicht Bevorzugt wird DS mit einem Molekulargewicht unter 40 kDa, ganz besonders bevorzugt mit einem MG von 10-30 kDa.

Die bevorzugten Konzentrationsbereiche für elektrolythaltige Polyole wie Dextransulfat sind 1-10%, besonders 1-6 %, ganz besonders 3-5%, bezogen auf die Gesamtmenge der eingesetzten Monomeren.

Durch die Variation der Menge des elektrolythaltigen Polyols im Polymerisationsgemisch des Extruders lassen sich dabei das Molekulargewicht und die inhärente Viskosität der erfindungsgemäßen Polyester bei ansonsten unveränderten Prozessbedingungen in besonders einfacher Weise steuern, wie Tabelle 1 zeigt: Mit steigender Konzentration zugesetzten DS sinkt das mittlere Molekulargewicht und die inhärente Viskosität.

| DS-Menge | Chargen Nummer | Inhärente Viskosität (ChCl3) | Mw | Sn (II) |
|---|---|---|---|---|
| 2% | RES-0085 | 0,38 dl/g | 28,8 kDa | 1200 ppm |
| 3% | RES-0106 | 0,30 dl/g | 21,1 kDa | 1200 ppm |
| 4% | RES-0083 | 0,24 dl/g | 14,3 kDa | 1200 ppm |

Bevorzugt werden erfindungsgemäß Polyol-modulierte PLG-Polyester mit einem mittleren Molekulargewicht von 7-40 kDa, besonders von 10-30 Kda, da diese PLG-Polyester zur Herstellung von Depotformen, insbesondere zur Herstellung von Mikropartikeln besonders geeignet sind und ein besonders vorteilhaftes Freisetzungsprofil aufweisen.

Unter dem "mittleren Molekulargewicht" eines Polyesters wird in dieser Patentanmeldung das Molekulargewicht (Mw) verstanden, das sich aus GPC-Analyse unter Verwendung von Polystyrolstandards zur Kalibrierung ergibt, wenn Tetrahydrofuran als Eluent verwendet wird.

In einer bevorzugten Ausführungsform der Erfindung hat eine 0,1%ige Lösung des erfindungsgemäß DS-modulierten PLG-Polyesters in Chloroform bei 25°C eine inhärente Viskosität von 0.2-0.45 dl/g und ein Molekulargewicht von 10-30 KDa.

Im Vergleich zu den aus dem nächsten Stand der Technik bekannten PLG-Polyester, die mit Hilfe des Modulators Milchsäure hergestellt wurden, haben die erfindungsgemäß DS-modulierten PLG-Polyester eine raschere Abbaurate, die dem gewünschten Profil der Wirkstofffreisetzung besser angepasst ist. Zudem kann eine Akkumulation des Polyesters verhindert werden.

Insgesamt wird der initiale Wirkstoff-"burst" deutlich reduziert und die dem "burst" folgende Phase stark verminderter Wirkstofffreisetzung erheblich verkürzt bzw. eliminiert (siehe Abbildung 1). Gleichzeitig wird die Gesamtfreisetzungsdauer verlängert, so daß in vivo eine kontinuierliche Wirkstofffreisetzung über einen Zeitraum von 2-3 Wochen erreicht wird (siehe Abbildungen 1-2).

Die erfindungsgemäßen PLG-Polyester sind löslich in verschiedenen organischen Lösungsmittel wie Benzol, Aceton und Chloroform. Die mittlere Blocklänge der erfindungsgemäß modulierten PLG-Polyesters liegt zwischen 2,5 und 4 und die Glasübergangstemperatur beträgt etwa 40°C.

Vorteilhafterweise lässt sich im Extruder ein kontinuierlicher Prozess mit definiertem termodynamischem Profil etabilieren, der in exzellenter Weise zur großtechnischen Produktion der erfindungsgemäßen Polyester geeignet ist. Dabei können in verschiedenen Zonen des Extruders unterschiedliche Temperaturen vorgegeben werden, so daß ein exakt definierbares Temperaturprofil bereitgestellt werden kann. Es stellte sich heraus, dass Polymerisationstemperaturen von 170-220°C, insbesondere von 170-200°C, zur Herstellung der erfindungsgemäßen Polyester besonders geeignet sind.

Geeignete Katalysatoren für die Ringöffnungs-Polymerisation von Hydroxycarbonsäuren sind Zinn, Zink-, Titan- oder Aluminiumverbindungen, wobei Zinn (II)-Verbindungen, wie Zink (II)-Oxid und insbesondere Zinn (II)-Octoat besonders bevorzugt werden.

Bevorzugte Katalysator-Konzentrationen zur Herstellung der erfindungsgemäßen Polyester sind Konzentrationen von über 1000 ppm, insbesondere 1000-2000 ppm und ganz besonders 1200-1600 ppm, bezogen auf die Gesamtmenge der eingesetzten Monomeren. Bei Zusatz eines Katalysatorgehalts unter 600 ppm sind die erfindungsgemäßen Polyester hingegen schlechter in organischen Lösungsmittel löslich, was die Aufarbeitung erschwert.

Da der Einsatz der hohen Katalysator-Konzentrationen toxikologisch nicht unbedenklich ist, wird der Metallionen-Gehalt des Polyesters nach abgeschlossener Polymerisation bevorzugt wieder abgereichert.

Ein bevorzugter Gegenstand der Erfindung ist daher ein Polyester, hergestellt durch die Ringöffnungs-Polymerisation von Hydroxycarbonsäuren in Gegenwart eines elektrolythaltigen Polyols im Extruder, wobei die Polymerisation unter Zinn (II)-Katalyse verläuft und der Zinn (II)-Gehalts des Polyesters auf unter 30 ppm, bevorzugt unter 20 ppm, abgereichert wird.

Die wesentlichen Unterschiede zwischen den aus dem Stand der Technik bekannten Milchsäure modulierten PLG-Polyestem und den erfindungsgemäß DS-modulierten PLG-Polyestern werden in Tabelle 2 zusammengefasst:

**Tabelle 2:**

| | **Milchsäure-modulierte Polyester** | **DS-modulierte Polyester** |
|---|---|---|
| Peptidfreisetzung | Sigmoid über maximal 14 Tage | Linear über 20-30 Tage |
| Abbau | Langsam | Schnell |
| Hydrophilität | Gering | Hoch |
| Katalysatormenge | Bis 600 ppm | Bevorzugt über 1000 ppm |

Ein weiterer Gegenstand der vorliegenden Erfindung sind Verfahren zur Herstellung der erfindungsgemäßen resorbierbaren Polyester.

Ein Gegenstand der Erfindung ist die Herstellung resorbierbarer Polyester durch Ringöffnungs-Polymerisation von Hydroxycarbonsäuren im Extruder, dadurch gekennzeichnet, dass dem Polymerisationsgemisch elektrolythaltige Polyole als Moderatoren zugesetzt werden.

Die Zugabe des elektrolythaltigen Polyols erfolgt bevorzugt vor der Zugabe des Katalysators. Der Moderator kann im Extruder vorgelegt werden, gemeinsam mit einem oder mehreren Monomeren gemischt in den Extruder gegeben werden oder zu den im Extruder vorgelegten und vorgemischten Monomeren gegeben werden. Alle diesbezüglichen Varianten sind Gegenstand der Erfindung und werden in dieser Patentanmeldung unter dem Ausdruck "dem Polymerisationsgemisch wird ein elektrolythaltiges Potyol/ein Moderator zugesetzt" subsumiert.

in einer bevorzugten Ausführungsform umfasst die Erfindung Verfahren zur Herstellung resorbierbarer Polyester durch Ringöffnungs-Polymerisation im Extruder, wobei als Monomere Lactid und/oder Glycolid zum Einsatz kommen und wobei DS als Moderator zugesetzt wird.

Wie bereits weiter oben ausgeführt wurde von den Erfindern überraschenderweise festgestellt, das der Einsatz hoher Katalysatormengen bei der Ringöffnungs-Polymerisation von Hydroxycarbonsäuren besonders vorteilhaft ist. Die entstehenden Polyester weisen bei der Zugabe höherer Katalysatormengen ein besseres Freisetzungsprofil auf und lassen sich besser aufarbeiten.

Ein weiterer Gegenstand der Erfindung ist daher ein Verfahren zur Herstellung von Polyhydroxycarbonsäuren durch Ringöffnungspolymersierung im Extruder, dadurch gekennzeichnet, dass dem Monomerengemisch als Katalysator mindestens 1000 ppm Sn (II), bevorzugt 1000-2000 ppm Sn (II), besonders bevorzugt 1200-1600 ppm Sn (II), bezogen auf die eingesetzte Menge Monomeren, zugesetzt werden.

Ferner kann das Verfahren die Entfernung des Katalysators umfassen. Entsprechende Reinigungsmethoden sind aus dem Stand der Technik bekannt. EP 270 987 beschreibt beispielsweise die Entfernung von Katalysator aus Polymeren durch Behandlung der Polymere mit Säuren oder EDTA in Zweiphasensystemen.

Alternativ kann die Entfernung des Katalysators auch in einem Einphasensystem erfolgen, indem das Polymer nach Zugabe von Säuren aus dem Lösungsmittelsystem abgetrennt wird. Bevorzugt werden zur Abtrennung des Katalysators Carbonsäuren, wie zB. Essigsäure, Malonsäure, Citronensäure oder Milchsäure verwendet. Durch dieses Verfahren kann der Zinn (II)-Gehalt regelmäßig auf unter 30 ppm, bevorzugt auf unter 20 ppm gesenkt werden.

in einer Ausführungsform umfasst das Herstellungsverfahren des erfindungsgemäßen resorbierbaren Polyester den Einsatz der folgenden Reagenzien im Extruder:
(a) Hydroxycarbonsäuren in Lactonform
(b) 1-10% eines elektrolythaltigen Polyols, bezogen auf die Gesamtmenge eingesetzter Hydroxycarbonsäuren
(c) Polymerisationskatalysator.

In einer besonders bevorzugten Ausführungsform umfasst das Herstellungsverfahren des erfindungsgemäßen resorbierbaren Polyester den Einsatz der folgenden Reagenzien im Extruder:
(a) Lactid/Glycolid im Verhältnis 3:1 bis 1:3, bevorzugt 60/40-40/60 und ganz besonders bevorzugt 55/45-45/55
(b) 1-6 %, bevorzugt 3-5% Dextransulfat, bezogen auf die Gesamt-Monomerenmenge
(c) mindestens 1000 ppm Zinn (II), bevorzugt 1000-2000 ppm Zinn (II), besonders bevor zugt 1200-1600 ppm, bezogen auf die Gesamt-Monomerenmenge,

Ein weiterer Gegenstand der Erfindung ist ein Verfahren zur Herstellung des erfindungsgemäßen resorbierbaren Polyesters, umfassend das Polymerisieren von Hydroxycarbonsäuren, dadurch gekennzeichnet, dass das Verfahren nacheinander die folgenden Schritte umfasst:
(a) Mischen der Hydroxycarbonsäuremonomere in Gegenwart von elektrolythaltigem Polyol
(b) Einfüllen der Mischung aus (a) in den Extruder
(c) Zugabe von Katalysator
(d) Polymerisieren im Extruder

Ein bevorzugter Gegenstand der Erfindung ist ein Verfahren zur Herstellung des erfindungsgemäßen resorbierbaren Polyesters, umfassend das Polymerisieren von Lactid und Glycolid, dadurch gekennzeichnet, dass das Verfahren nacheinander die folgenden Schritte umfasst:
(a) Mischen von Lactid und Glycolid im Verhältnis 60/40-40/60 in Gegenwart von 1-6% DS, bezogen auf die Gesamtmonomerenmenge
(b) Einfüllen der Mischung aus (a) in den Extruder
(c) Zugabe von 1.000 - 2000 ppm Zinn (II)
(d) Polymerisieren im Extruder bei 170-220°C,

In diesen Ausführungsformen der Erfindung ist es nicht kritisch, in welcher Reihenfolge die in Schritt (a) gemischten Komponenten zusammengegeben werden. So können beispielsweise Lactid, Glycolid und DS gleichzeitig oder sukzessive in den Extruder eingefüllt werden. Alternativ kann eine Vormischung zweier oder mehrerer Komponenten, z.B. Lactid und DS, hergestellt werden, zu der dann eine weitere Komponente z.B. das Glycolid gegeben wird. Alle diesbezüglich möglichen Varianten sind Gegenstand des Schutzbereichs und werden unter dem Ausdruck "Mischen der Hydroxycarbonsäure-Monomere in Gegenwart von elektrolythaltigem Polyol" subsumiert.

Durch den erfindungsgemäßen Zusatz von elektrolythaltigen Polyolen, besonders bevorzugt von DS, bei der Polymerisation im Extruder können die Eigenschaften von resorbierbaren Polyestern in besonders vorteilhafter und einfacher Weise gesteuert werden.

Ein weiterer Gegenstand der vorliegenden Erfindung ist daher die Verwendung eines elektrolythaltigen Polyols bei der Herstellung eines resorbierbaren Polyesters im Extruder.

In einer bevorzugten Ausführungsform wird als elektrolythaltiger Polyol bei der Herstellung eines resorbierbaren Polyesters im Extruder DS verwendet.

Die erfindungsgemäßen Polyester sind insbesondere zur Verwendung als Matrixpolymere für Depotformen pharmakologisch aktiver Wirkstoffe geeignet Beispiele solcher Depotformen sind besonders parenterale Arzneiformen wie Mikropartikel, Kapseln, Implantate, Pulver, Granulate oder Pellets aber auch inhalative oder topische Arzneiformen, wie z.B. bioabbaubare Wundfolien oder PLGA-Nanospheren zur pulmonalen Applikation.

Ein Gegenstand der Erfindung ist daher eine pharmazeutische Formulierung, enthaltend einen erfindungsgemäßen Polyester und mindestens einen pharmakologisch aktiven Wirkstoff.

Dabei kann der Polyester als Polyester vorliegen, in das der Wirkstoff eingebettet (dispergiert oder gelöst) ist, der Polyester kann den Wirkstoff umhüllen oder der Polyester kann an der Oberfläche mit Wirkstoff beschichtet sein.

In einer bevorzugten Ausführungsform liegt der Polyester in Form von Mikropartikeln vor, in das der Wirkstoff eingebettet ist. Die Herstellung solcher Mikropartikel ist aus dem Stand der Technik bekannt. Gängige Techniken sind z.B. Koazervation, Lösungsmittelextraktion aus einer Öl-Wasser Dispersion oder Sprühtrocknung.

Ein Gegenstand der Erfindung ist daher eine pharmazeutische Formulierung, enthaltend einen erfindungsgemäßen Polyester und mindestens einen pharmakologischen Wirkstoff, wobei das Polyester und der Wirkstoff in Mikropartikeln vorliegen.

Die erfindungsgemäßen Formulierungen sind generell zur Verabreichung pharmakologisch aktiver Wirkstoffe geeignet. Von besonderem Interesse sind die Formulierungen zur Verabreichung von biologisch aktiven Peptiden und Proteinen, da diese in der Regel oral nicht oder unzureichend wirksam sind.

Nicht-limitierende Beispiele für solche Peptide sind Oxytocin; Vasopressin; adrenocorticotropes Hormon (ACTH); Wachstumsfaktoren wie z.B. epidermale Wachstumsfaktoren (EGF), Fibroblasten-Wachstumsfaktoren (a-FGF, b-FGF, FGF-9 etc), vascular endothelial growth factor (VEGF), Tumor Nekrose Faktor (TNF), Plättchen-Wachstumsfaktor (PDGF), Neurotrophine (NT 1-3, BDNF, NGF), Erythropoetin oder Insulin-ähnliche Wachstumsfaktoren (IGF); Releasing Faktoren wie z.B. luteinisierendes hormon releasing Hormon (LHRH), Wachstumshormon-releasing Faktor (GRF), Gonadotropin-releasing Hormon (GnRH), Gastrin-reteasing factor oder Tyrosin-releasing Hormon (TRH); Thyroidstimulierendes Hormon (TSH); Parathyroid Hormon (PTH), luteinisierendes Hormon (LH); Follikel-stimulierendes Hormon (FSH); Somatostatin und Analoga; Somatotropin; Gastrin; Prolactin; Motilin; Kallikrein; Amylin; Glucagon; glucagon like peptide (GLP); Calcitionin; calcitonin-related peptide; natriuretische Proteine; Angiotensine; Renin; Brandykinin; Enkephaline; Endorphine; Interferone (Alpha, Beta, Gamma); Chemokine; Hematopoetische Wachstumsfaktoren wie z.B. Erythropoetin; Stammzellenwachstumsfaktor (SCF), lnterleukine (z.B. IL-1 bis IL12), Granulozytenwachstumsfaktoren (G-CSF und GM-CSF) oder Monozytenwachstumsfaktoren (M-CSF); Peptidantibiotika wie z.B. Tyrocidin, Gramicidin, Bacitracin oder Nisin; Angiopeptin; Hirudin; Thrombopoetin, Urogastrone; Knochenbildende Proteine wie das bone morphogenic protein, Antikörper sowie deren Fragmente und Abkömmlinge (Fab, (Fab)₂, Diabodies, scFVs etc.); Transkriptionsfaktoren; Peptidnucleinsäuren; Vaccin-Peptide viraler oder mikrobieller Herkunft; tumor-basierte Peptide wie z.B. PSA, PSMA, PSCA; HLA-Peptide bzw. MHC-Antigene; Leukozyten-Marker (z.B. CD3, CD 11 a-c, CD28, CD30, Cdw52) und ihre Liganden (z.B. B7); T-Zell-Rezeptoren und deren Fragmente; angiostatische Peptide wie Angiostatin oder Endostatin; Onconase; Integrine und Integrin-inhibierende Peptide (RGDS-Peptide); Lektine wie z.B. Mistel-Lektin; Calmodulin; vacoactive intestinal peptide (VIP); Fertilization Promoting Peptide (FPP); Cocaine and amphetamine regulated transcript peptide (CART); Leptin und Derivate; Lösliche Rezeptoren; Endothelin; Insulin; Proinsulin und C-Peptid sowie deren biologisch aktive Varianten und Fragmente.

Dabei werden Formulierungen bevorzugt, die als Depotform ausgebildet sind. Als "Depotform" werden hier pharmazeutische Zubereitungen verstanden, die den Wirkstoff über einen Zeitraum von mindestens drei Tagen, bevorzugt zehn Tagen, ganz bevorzugt von 2, 3 oder 4 Wochen in therapeutisch wirksamer Menge freisetzen.

Ein besonders bevorzugtes Peptid ist das humane Proinsulin C-Peptid, ein 31-Aminosäuren-Peptid aus dem Proinsulin mit therapeutischer Wirksamkeit bei der Behandlung von Diabetes und diabetischer Folgeerkrankungen sowie deren Fragmente und Derivate wie in der OS WO 98/13384 beschrieben.

Schließlich ist ein weiterer Gegenstand der vorliegenden Erfindung ein Kit umfassend eine pharmazeutische Formulierung wie oben beschrieben und eine Vorrichtung zur Verabreichung der Formulierung. Diese kann als Spritze oder nadellose Injektionsvorrichtung ausgebildet sein. Entsprechende Beispiele sind im Stand der Technik beschrieben und dem Fachmann bekannt.

Ein solches Kit kann auch eine Vorrichtung zur Resuspension der pharmazeutischen Formulierung umfassen, wenn die Formulierung in trockener Form vorliegt und unmittelbar vor der Applikation gelöst oder resuspendiert werden muß.

### Ausführungsbeispiele:

### Beispiel 1: Herstellung eines DS-Polyesters im Extruder

### Polymerisation:

Zunächst wird das DS mind. 3 Tage bei Raumtemperatur im Feinvakuum (ca. 0,1 mbar) getrocknet.

In ein Mischfaß werden 53 mol% D,L-Lactid, 47 mol% Glycolid und das vorgetrocknete DS (2 bis 4 Gew.%) eingewogen. Zur Homogenisierung wird 30 min in einem Rhönradmischer gemischt.

Die Monomer/DS-Vormischung wird in eine Dosierwaage des Extruders (Leistritz Doppelschnecken-Extruder typ LSM 34 GG) eingefüllt und dann kontinuierlich über Dosierschnecken in den Extruder eindosiert.

Eine definierte Menge des Katalysator Zinn(II) 2-ethylhexanoat wird in Toluol gelöst und diese Lösung dann mittels einer HPLC Pumpe kontinuierlich in den Extruder eindosiert. Die Förderrate wird unter Berücksichtiugung der Feedrate so eingestellt, dass die Katalysatormenge in der Reaktionsmischung 1200 ppm beträgt

Die Polymerisation wird sodann im Extruder bei Temperaturen zwischen 170-220 °C durchgeführt..

### Aufarbeitung

Das Rohpolymer wird über Nacht in Aceton gelöst. Nach vollständigem Lösen wird D,L-Milchsäure zugesetzt und weitere 2-3 h gerührt. Die erhaltene trübe Lösung (10 Gew.% Polyester in Lösungsmittelgemisch) wird mit leichtem Stickstoffdruck abfiltriert.

Die filtrierte Polyesterlösung wird mit entmineralisiertem Wasser gefällt. Das ausgefallene Polyester wird nachgewaschen, gefiltert und anschließend bei max. 35°C bis zur Gewichtskonstanz getrocknet.

### Beispiel 2: Polyester- Analyse

DS-Polyester, die wie in Beispiel 1 beschrieben hergestellt wurden, wurden im Hinblick auf ihre chemische Zusammensetzung, ihr Molekulargewicht und andere charakteristische Kennzahlen analysiert und mit kommerziellen PLG-Standardpolymeren verglichen. Das Resultat wird in der nachstehenden Tabelle wiedergegeben:

| **Prüfpunkt** | **DSS-PLG 3% DSS, (1600ppm Sn)** | **DSS-PLG** 4% DSS, **(1600ppm Sn)** | **DSS-PLG 3% DSS, (1200ppm Sn)** | **Resomer RG502H** | **Resomer RG503H** |
|---|---|---|---|---|---|
| **M**_{**w**} **[g/mol]** | 17234 | 14137 | 21056 | 8859 | 26151 |
| **M**_{**n**} **[g/mol]** | 9486 | 7952 | 8493 | 5476 | 11469 |
| **PD [M**_{**w**}**/M**_{**n**}**]** | 1,8 | 1,8 | 2,5 | 1,6 | 2,3 |
| **IV [dL/g]** | 0,25 | 0,22 | 0,30 | 0,21 | 0,35 |
| **T**_{**g**} **[°C]** | 39,6 | 40,5 | 38,9 | 38,0 | 41,0 |
| **Lac : Glyc** | 53,2 : 46,8 | 53,1 : 46,9 | 51,0 : 49,0 | 52,5 : 47,5 | 52,2 : 47,8 |
| **Blocklänge** | 3,0 | 3,4 | | | |
| **KH-Gehalt [ppm]** | 13 | 25 | | < NWG | < NWG |
| **Säurezahl [mg KOH/g]** | 7,9 | 9,0 | 6,7 | 12,6 | 4,2 |
| **Wassergehalt [%]** | 0,4 | 0,5 | | 0,4 | 0,4 |
| **Sulfatasehe[%]** | 0,0 | 0,0 | | 0,0 | 0,0 |
| **Sn-Gehalt [ppm]** | 13 | 14 | < 10 | 145 | 112 |
| **Na-Gehalt [ppm]** | < 5 | < 5 | < 5 | < 5 | < 5 |
| **S-Gehalt [ppm]** | < 5 | < 5 | < 5 | < 5 | < 5 |
| M_{w}: Molekulargewicht (Gewichtsmittel), Mₙ: Molekulargewicht (Zahlenmittel), PD: Polydispersität, IV: inhärente Viskosität, Lac: Glyc: molares Verhältnis Lactid zu Glycolid, KH: Kohlenhydratanteil, Sn: Zinn, Na: Natrium, S: Schwefel, NWG: Nachweisgrenze | | | | | |

### Beschreibung der analytischen Verfahren:

### Molekulargewichtsbestimmung:

Die Molekulargewichtsdaten (M_{w}, Mₙ, PD) wurden mittels Gelpermeationschromatographie (GPC) auf Ultrastyragel-Säulen unter Verwendung einer Polystyrol-Kalibrierung bestimmt. Als mobile Phase diente Tetrahydrofuran (THF). Die Detektion erfolgte mittels RI und UV.

### Inhärente Viskosität (IV)

Die inhärenten Viskositäten werden mittels eines Schott Ubbelohde Viskosimeters (gemäß DIN 51562) mit der Kapillargröße 0 bestimmt. Probelösung ist eine 0,1%ige Lösung des Polymers in Chloroform. Die Messtemperatur beträgt 25°C.

### Glasübergangstemperatur (Tg)

Die Glasübergangtemperatur Tg wird mittels differential scanning calorimetrie (DSC) ermittelt.

### Lactid-Glycolid Verhältnis

Das Lactid-Glycolid Verhältnis der Copolymere wird mittels ¹H-NMR Spektroskopie bestimmt. Dazu werden 5 - 20 mg der zu analysierenden Substanz in ca. 0,6 mL Deuterochloroform gelöst und ¹H-NMR Spektren an einem 200 MHz Spektrometer bei einer Temperatur von 25°C aufgenommen. Als interner Standard dient Tetramethylsilan. Die Berechnung des Lactid-Glycolid-Verhältnisses wird durch quantitative Bestimmung der Integrale der Lactideinheiten (multiplett bei ca. 5,2 ppm) sowie der Glycolideinheiten (mulitplett bei ca. 4,8 ppm) durchgeführt.

### Bestimmung der Blocklänge

Die Bestimmung der mittleren Blocklänge wird mittels ¹³C-NMR spektroskopischer Untersuchung durchgeführt.

### Kohlenhydratgehalt

Zur Bestimmung des Gesamtkohlenhydratgehaltes der Polymere werden die Polymere in 12molarer Schwefelsäure hydrolysiert. Die quantitative Bestimmung als Glukose erfolgt nach anionenaustauschchromatographischer Trennung und anschließender elektrochemischen Detektion bezogen auf eine Kalibrierung mit einer Glukose-Referenzlösung.

### Wassergehalt

Die Bestimmung des Wassergehaltes erfolgt mittels coulometrischer Karl-Fischer Titration nach Ausheizen des Wassers im Trockenofen.

### Zinn-, Natrium- und Schwefelgehalt

Die Bestimmung von Zinn, Natrium und Schwefel erfolgt mittels ICP-Atomemissionsspektroskopie nach Druckaufschluss der Polymere in einer Mischung aus Salpetersäure und Flusssäure.

### Beispiel 3: Zinn (II)-Entfernung

40 kg Polymer wird in einem 1.000 I Rührwerksapparat vorgelegt. Anschließend wird 390 1 Aceton zugesetzt. Das Polymer wird unter Rühren gelöst. Anschließend wird 54 kg (45 I) D,L-Milchsäure zugesetzt und die Lösung weitere 2-3 h gerührt.

Die Lösung wird über ein 5µ-Filter vorfiltriert, in die Fällungsapparatur (Zweistoffdüse) gepumpt und darin mit entmineralisiertem Wasser versetzt. Nach der Fällung wird das Wasser-Aceton-Gemisch über ein Siphon abgelassen und das Polymer mit Wasser gewaschen. Sodann wird das Polymer bei maximal 30°C mit einer Alexanderreibe über ein Sieb zerkleinert und schließlich auf einem Filtertrockner vom Wasser befreit.

### Beispiel 4: Mikropartikelherstellung

0,55 g Polymer, bzw. Polymermischung wurde jeweils in 6 mL Eisessig gelöst, 0,045 g C-Peptid in 0,15 mL Wasser und 3,o mL Eisessig gelöst und langsam in die Polymerlösung eingelöst. Die Lösung wird in einem Büchi 190 Sprühtrockner bei 60°C versprüht und getrocknet, bis die Mikropartikel als feines, rieselfähiges Pulver gewonnen werden können.

### Beispiel 5: Pharmazeutische Retardformutierung C-Peptid

300 mg Mikropartikel, die 22,5 mg C-Peptid Acetat (Polypeptide Laboratories) enthielten werden in 1,5 mL Lösungsmittel (0,9% Kochsalz, 0,1%Tween) resuspendiert. Die erhaltene Suspension wird dem Patienten nach gründlichem Aufschütteln appliziert.

### Beispiel 6: in vivo Freisetzungsrate DS-Polyester in Vergleich zu Milchsäuremodulierten kommerziellen Polyestern

Beagle-Hunden wurde subkutan jeweils 300 mg Mikropartikel, beladen mit 7,5% C-Peptid, und basierend entweder auf DS-modulierten Polymeren oder auf käuflichen Standardpolymeren, appliziert. Zu den angegebenen Zeiten wurde den Tieren Blut angenommen und der C-Peptid-Plasmaspiegel mittels LC-MS quantifiziert. Die Resultate werden in den Abbildungen 1 und 2 dargestellt.

## Patentansprüche

1. Resorbierbarer Polyester, hergestellt durch Ringöffnungspolymerisierung von in Lactonform vorliegenden Hydroxycarbonsäuren in Gegenwart eines elektrolythaltigen Polyols, **dadurch gekennzeichnet, daß** die Ringöffnungspolymerisierung bei Temperaturen von 170 - 220°C im Extruder stattfindet und der Polyester zu weniger als 200 ppm elektrolythaltiges Polyol enthält.

2. Resorbierbarer Polyester nach Anspruch 1, **dadurch gekennzeichnet, dass** die Ringöffnungspolymerisierung bei einer Temperatur von 170-200 °C stattfindet.

3. Resorbierbarer Polyester nach einem der vorhergehenden Ansprüche, **dadurch gekennzeichnet, daß** die Ringöffnungspolymerisierung in Gegenwart von 1000-2000 ppm Sn (II), bezogen auf die eingesetzte Menge Monomeren durchgeführt wird.

4. Resorbierbarer Polyester nach einem der vorhergehenden Ansprüche, **dadurch gekennzeichnet, daß** der Polyester ein Molekulargewicht von 7-40 Kda aufweist.

5. Resorbierbarer Polyester nach einem der vorhergehenden Ansprüche, wobei die Hydroxycarbonsäuren Lactid und/oder Glycolid sind.

6. Resorbierbarer Polyester nach einem der vorhergehenden Ansprüche, wobei der elektrolythaltige Polyol Dextransulfat ist.

7. Resorbierbarer Polyester nach einem der vorhergehenden Ansprüche, wobei als elektrolythaltiges Polyol Dextransulfat in einer Konzentration von 1-6 % (w/w), bezogen auf die eingesetzte Menge Monomeren, zugesetzt wird.

8. Resorbierbarer Polyester nach einem der vorhergehenden Ansprüche, weiterhin **dadurch gekennzeichnet, daß** der resorbierbare Polyester eine inhärente Viskosität von 0,25-0,45 dl/g (0.1 % in Chloroform, 25°C) hat.

9. Verfahren zur Herstellung eines Polyesters nach einem der vorhergehenden Ansprüche.

10. Verfahren zur Herstellung eines resorbierbaren Polyesters gemäß einem der Ansprüche 1-8, wobei das Verfahren die folgenden Schritte umfaßt:
a) Mischen der in Lactonform vorliegenden Hydroxycarbonsäuren mit elektrolythaltigem Polyol
b) Einfüllen der Mischung aus (a) in einen Extruder
c) Zugabe von Katalysator
d) Polymerisieren im Extruder bei Temperaturen von 170 - 220°C.

11. Verfahren nach Anspruch 10, wobei der Katalysator in Konzentrationen von mindestens 1000 ppm bezogen auf die Gesamtmenge der entsprechenden Hydroxycarbonsäure-Monomere zugesetzt wird

12. Verfahren nach einem der vorhergehenden Ansprüche, wobei das elektrolythaltige Polyol in Schritt (a) in einer Konzentration von 1-6 % (w/w), bezogen auf die eingesetzte Menge Monomeren, zugesetzt wird.

13. Verfahren nach einem der vorangegangenen Ansprüche, ferner umfassend die Abreicherung des Katalysatorgehalts im Polymer auf einen Wert unter 30 ppm.

14. Verfahren nach einem der vorhergehenden Ansprüche, wobei die Hydroxycarbonsäuren Glycolid- und/oder Lactid-Einheiten sind.

15. Verfahren nach einem der vorhergehenden Ansprüche, wobei der elektrolythaltige Polyol Dextransulfat ist.

16. Verwendung eines resorbierbaren Polyesters nach einem der vorhergehenden Ansprüche zur Herstellung eines Arzneimittels.

17. Pharmazeutische Formulierung umfassend einen resorbierbaren Polyester nach einem der vorhergehenden Ansprüche und einen pharmakologisch aktiven Wirkstoff.

18. Pharmazeutische Formulierung nach Anspruch 17, **dadurch gekennzeichnet, dass** der resorbierbare Polyester in Form von Mikropartikeln vorliegt.

19. Pharmazeutische Formutierung nach einem der vorhergehenden Ansprüche, wobei der Wirkstoff ein Peptid ist.

20. Pharmazeutische Formulierung nach einem der vorhergehenden Ansprüche, wobei das Peptid über einen Zeitraum von mindestens zwei Wochen in einer therapeutisch wirksamen Menge freigesetzt wird.

## Claims

1. An absorbable polyester, obtainable by ring-opening polymerizing hydroxycarboxylic acids in lactone form in the presence of an electrolyte-containing polyol, wherein ring-opening polymerizing occurs at a temperature of from 170 to 220°C in the extruder and the polyester contains less than about 200 ppm of the electrolyte-containing polyol.

2. The absorbable polyester of claim 1 wherein the ring-opening polymerizing takes place at a temperature of from 170 to 200°C.

3. The absorbable polyester of one of the preceding claims wherein the ring-opening polymerizing occurs in the presence of from 1000 to 2000 ppm Sn (II) relative to the amount of monomer used.

4. The absorbable polyester of one of the preceding claims wherein the polyester has a molecular weight of 7 to 40 kDa.

5. The absorbable polyester of one of the preceding claims wherein the hydroxycarboxylic acids are lactides and/or glycolides.

6. The absorbable polyester of one of the preceding claims wherein the electrolyte-containing polyol is dextran sulfate.

7. The absorbable polyester of one of the preceding claims wherein the electrolyte-containing polyol dextran sulfate is used at a concentration of 1 to 6 percent (w/w) relative to the amount of monomer.

8. The absorbable polyester of one of the preceding claims wherein the absorbable polyester has an inherent viscosity of 0.25 to 0.45 dl/g (0.1 % in chloroform, 25°C).

9. A method for producing a polyester of one of the preceding claims.

10. Method for producing an absorbable polyester according to one of the claims 1-8, wherein the method comprises the following steps:
a) mixing the lactone-form hydroxycarboxylic acids with electrolyte-containing polyol;
b) introducing the mixture of a) into an extruder;
c) adding a catalyst;
d) polymerizing in the extruder at a temperature of from 170 to 220°C.

11. The method of claim 10 wherein the catalyst is added at a concentration of at least 1000 ppm relative to the total amount of the respective hydroxycarboxylic acid monomer.

12. The method of one of the preceding claims wherein in a) the electrolyte-containing polyol is added at a concentration of 1 to 6 percent (w/w) relative to the amount of monomer used.

13. The method of one of the preceding claims further comprising the depletion of the catalyst content in the polymer to below 30 ppm.

14. The method of one of the preceding claims wherein the hydroxycarboxylic acids are lactide- and/or glycolide-monomers.

15. The method of one of the preceding claims wherein the electrolyte-containing polyol is dextran sulfate.

16. Use of an absorbable polyester of one of the preceding claims for producing a medicament.

17. Pharmaceutical formulation comprising an absorbable polyester of one of the preceding claims and a pharmaceutically active substance.

18. The pharmaceutical formulation of claim 17 wherein the absorbable polyester is in the form of microparticles.

19. The pharmaceutical formulation of one of the preceding claims wherein the active substance is a peptide.

20. The pharmaceutical formulation of one of the preceding claims wherein the peptide is released in an therapeutically effective quantity over a period of at least two weeks.

## Revendications

1. Polyester résorbable, fabriqué par polymérisation par ouverture de cycle d'acides hydroxycarboxyliques présents sous forme de lactone en présence d'un polyol contenant un électrolyte, ***caractérisé en ce que*** la polymérisation par ouverture de cycle est conduite dans une extrudeuse à une température de 170-220°C et le polyester contient moins de 200 ppm de polyol à électrolyte.

2. Polyester résorbable selon la revendication 1, ***caractérisé en ce que*** la polymérisation par ouverture de cycle est conduite à une température de 170-200°C.

3. Polyester résorbable selon l'une quelconque des revendications précédentes, ***caractérisé en ce que*** la polymérisation par ouverture de cycle est conduite en présence de 1000-2000 ppm de Sn (II), rapporté à la quantité employée de monomères.

4. Polyester résorbable selon l'une quelconque des revendications précédentes, ***caractérisé en ce que*** le polyester présente un poids moléculaire de 7-40 Kda.

5. Polyester résorbable selon l'une quelconque des revendications précédentes, dans lequel les acides hydroxycarboxyliques sont le lactide et/ou le glycolide.

6. Polyester résorbable selon l'une quelconque des revendications précédentes, dans lequel le polyol contenant un électrolyte est le sulfate de dextrane.

7. Polyester résorbable selon l'une quelconque des revendications précédentes, dans lequel, comme polyol contenant un électrolyte, on ajoute du sulfate de dextrane selon une concentration de 1-6 % (poids/poids), rapporté à la quantité employée de monomères.

8. Polyester résorbable selon l'une quelconque des revendications précédentes, ***caractérisé en ce que*** le polyester résorbable présente une viscosité inhérente de 0,25 - 0,45 dl/g (0,1 % dans le chloroforme, 25°C).

9. Procédé de fabrication d'un polyester selon l'une quelconque des revendications précédentes.

10. Procédé de fabrication d'un polyester résorbable selon l'une quelconque des revendications 1 à 8, dans lequel le procédé comprend les étapes suivantes :
a) mélange des acides hydroxycarboxyliques présents sous forme de lactone avec un polyol contenant un électrolyte,
b) introduction du mélange de (a) dans une extrudeuse,
c) ajout d'un catalyseur,
d) polymérisation dans l'extrudeuse à des températures de 170-220°C.

11. Procédé selon la revendication 10,dans lequel le catalyseur est ajouté selon des concentrations d'au moins 1000 ppm rapporté à la quantité totale des monomères d'acides hydroxycarboxyliques.

12. Procédé selon l'une quelconque des revendications précédentes, dans lequel le polyol contenant un électrolyte de l'étape (a) est ajouté selon une concentration de 1-6 % en poids, rapporté à la quantité utilisée de monomères.

13. Procédé selon l'une quelconque des revendications précédentes, comprenant de plus la réduction de la teneur en catalyseur dans le polymère à une valeur inférieure à 30 ppm.

14. Procédé selon l'une quelconque des revendications précédentes, dans lequel les acides hydroxycarboxyliques sont le lactide et/ou le glycolide.

15. Procédé selon l'une quelconque des revendications précédentes, dans lequel le polyol contenant un électrolyte est le sulfate de dextrane.

16. Utilisation d'un polyester résorbable selon l'une quelconque des revendications précédentes pour fabriquer un médicament.

17. Formulation pharmaceutique contenant un polyester résorbable selon l'une quelconque des revendications précédentes et une substance pharmacologiquement active.

18. Formulation pharmaceutique selon la revendication 17, ***caractérisée en ce que*** le polyester résorbable est présent sous forme de microparticules.

19. Formulation pharmaceutique selon l'une quelconque des revendications précédentes, dans lequel la substance active est un peptide.

20. Formulation pharmaceutique selon l'une quelconque des revendications précédentes, dans lequel le peptide est libéré sur une période d'au moins deux semaines en une quantité thérapeutiquement active.
